(19) Europäisches Patentamt
European Patent Office
Office européen des brevets

(11) Veröffentlichungsnummer: **0 610 651 A1**

# (12) EUROPÄISCHE PATENTANMELDUNG

(21) Anmeldenummer: **94100100.0**

(22) Anmeldetag: **05.01.94**

(51) Int. Cl.$^5$: **C07D 207/26**, C07D 487/04, A01N 43/36, A01N 43/90, //(C07D487/04,207:26,207:26)

(30) Priorität: **18.01.93 DE 4301112**

(43) Veröffentlichungstag der Anmeldung:
**17.08.94 Patentblatt 94/33**

(84) Benannte Vertragsstaaten:
**CH DE ES FR GB IT LI**

(71) Anmelder: **BAYER AG**
**D-51368 Leverkusen (DE)**

(72) Erfinder: **Negele, Michael, Dr.**
**Ratinger Weg 8**
**D-42697 Solingen (DE)**
Erfinder: **Lui, Norbert, Dr.**
**Roggendorfstrasse 55**
**D-51061 Köln (DE)**
Erfinder: **Baasner, Bernd, Dr.**
**Wagnerstrasse 83**
**D-51467 Bergisch Gladbach (DE)**
Erfinder: **Hänssler, Gerd, Dr.**
**Am Arenzberg 58A**
**D-51381 Leverkusen (DE)**

## (54) N-substituierte alpha-Fluoralkyl-Lactame.

(57) Die neuen N-substituierten α-Fluoralkyl-Lactame der allgemeinen Formel

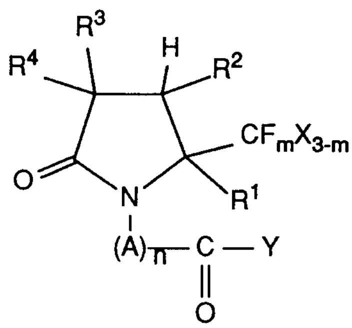

(I),

worin die Substituenten und Indices die in der Beschreibung angegebene Bedeutung besitzen, weisen eine starke Wirkung gegen Schädlinge auf und können insbesondere zur Bekämpfung unerwünschter Pilze eingesetzt werden.

EP 0 610 651 A1

Rank Xerox (UK) Business Services
(3.10/3.09/3.3.4)

Die vorliegende Erfindung betrifft neue N-substituierte α-Fluoralkyl-Lactame, neue Zwischenprodukte, ein Verfahren zur Herstellung der neuen N-substituierten α-Fluoralkyl-Lactame und deren Verwendung als Schädlingsbekämpfungsmittel.

Die neuen N-substituierten α-Fluoralkyl-Lactame entsprechen der allgemeinen Formel

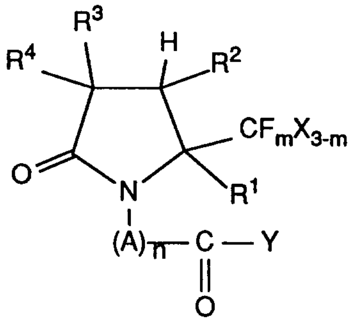

(I),

worin

| | |
|---|---|
| $R^1$ | für Wasserstoff, Halogen, Cyano, Alkyl, Aryl oder Alkoxycarbonyl steht, |
| $R^2$ und $R^3$ | unabhängig voneinander für Wasserstoff, Halogen, Alkyl oder Aryl stehen, |
| $R^4$ | für Wasserstoff, Halogen, Cyano, Alkyl, Aryl oder Alkoxycarbonyl steht, |
| A | für Alkylen steht, |
| Y | für Alkoxy, Amino, (Di)alkylamino oder Aryl steht, |

oder

| | |
|---|---|
| Y | gemeinsam mit dem Rest $R^1$ für Alkylen steht, |
| n | für 0 oder 1 steht, |
| X | für Wasserstoff, Halogen oder Alkyl steht, und |
| m | für 1, 2 oder 3 steht. |

Alle Alkylreste können geradkettig oder verzweigt sein. Weiterhin können alle Alkyl, Alkoxy, Aryl und Alkylenreste, auch wenn Sie in zusammengesetzten Begriffen wie z.B. Alkoxycarbonyl vorkommen, gegebenenfalls substituiert sein.

Die neuen N-substituierten α-Fluoralkyl-Lactame der Formel (Ia)

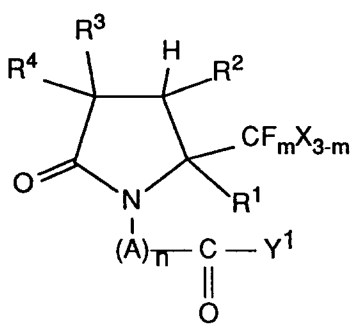

(Ia),

worin

| | |
|---|---|
| $R^1$, $R^2$, $R^3$, $R^4$, A, X, m und n | die für Formel (I) angegebene Bedeutung haben, und |
| $Y^1$ | für Alkoxy oder Aryl steht, |

können hergestellt werden durch Umsetzung von Pyrrolidinon-Derivaten der Formel

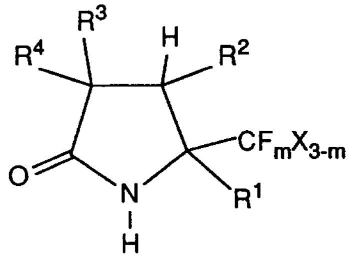

(II),

worin

$R^1$, $R^2$, $R^3$, $R^4$, X und m die oben angegebene Bedeutung haben

mit Verbindungen der Formel

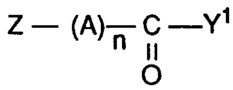

(III),

in welcher

Z für Halogen, insbesondere für Brom oder Chlor steht, und

A, $Y^1$ und n die oben angegebene Bedeutung haben,

gegebenenfalls in Gegenwart eines Verdünnungsmittels und gegebenenfalls in Gegenwart eines Säurebindemittels.

Die neuen N-substituierten α-Fluoralkyl-Lactame der Formel (Ib)

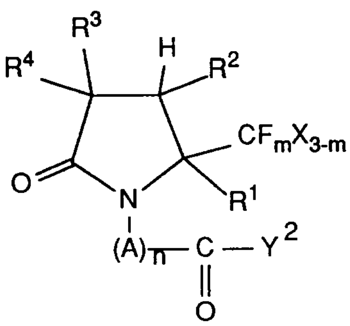

(Ib),

worin

$R^2$, $R^3$, $R^4$, A, X, m und n die für Formel (I) angegebene Bedeutung haben, und

$Y^2$ gemeinsam mit dem Rest $R^1$ für Alkylen steht,

können hergestellt werden durch Umsetzung von Verbindungen der Formel

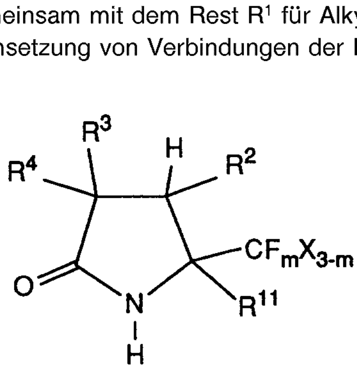

(IV),

worin

$R^{11}$ für gegebenenfalls substituiertes Alkoxycarbonylalkyl, vorzugsweise für gegebenenfalls substituiertes $C_1$-$C_4$-Alkoxycarbonyl-$C_1$-$C_4$-alkyl steht, und

$R^2$, $R^3$, $R^4$, X und m die oben angegebenen Bedeutung haben,

mit einer Base, vorzugsweise mit Alkalihydroxid und anschließender Umsetzung des erhaltenen Produktes

mit einem Wasser bindenden Mittel wie beispielsweise einem Säureanhydrid, vorzugsweise Essigsäureanhydrid, jeweils gegebenenfalls in Gegenwart eines Verdünnungsmittels.

Die neuen N-substituierten α-Fluoralkyl-Lactame der Formel (Ic)

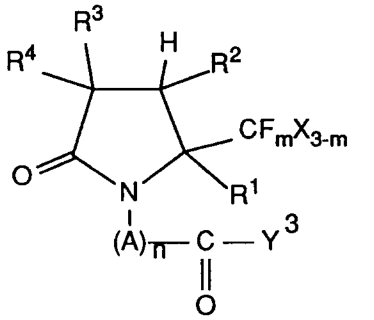
(Ic),

worin

| | |
|---|---|
| $R^1$, $R^2$, $R^3$, $R^4$, A, X, n und m | die für Formel (I) angegebene Bedeutung haben, und |
| $Y^3$ | für Amino oder (Di)alkylamino, vorzugsweise für Amino, $C_1$-$C_4$-Alkylamino oder $C_1$-$C_4$-Dialkylamino, welches jeweils gegebenenfalls durch (Di)-$C_1$-$C_4$-alkylamino substituiert ist, steht, |

können hergestellt werden durch Umsetzung von Verbindungen der Formel (Id)

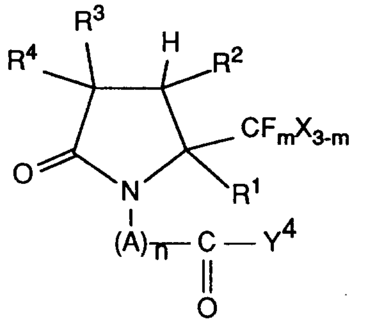
(Id),

worin

| | |
|---|---|
| $R^1$, $R^2$, $R^3$, $R^4$, A, X, n und m | die für die Formel (I) angegebene Bedeutung haben, und |
| $Y^4$ | für Alkoxy, vorzugsweise $C_1$-$C_4$-Alkoxy steht, |

mit einem Amin der Formel (V)

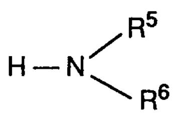
(V),

worin

| | |
|---|---|
| $R^5$ und $R^6$ | unabhängig voneinander für Wasserstoff oder $C_1$-$C_4$-Alkyl, welches gegebenenfalls durch (Di)-$C_1$-$C_4$-alkylamino substituiert ist, stehen. |

Schließlich wurde gefunden, daß die neuen N-substituierten α-Fluoralkyl-Lactame eine gute Wirksamkeit als Fungizide besitzen.

Die erfindungsgemäßen neuen N-substituierten α-Fluoralkyl-Lactame sind durch die Formel (I) allgemein definiert Bevorzugt sind Verbindungen der Formel (I), in welchen

| | |
|---|---|
| $R^1$ | für Wasserstoff, Fluor, Chlor, Brom, gegebenenfalls substituiertes $C_1$-$C_6$-Alkyl, gegebenenfalls substituiertes $C_6$-$C_{10}$-Aryl oder gegebenenfalls substituiertes $C_1$-$C_6$-Alkoxycarbonyl-($C_1$-$C_4$)-alkyl steht, |
| $R^2$ und $R^3$ | unabhängig voneinander für Wasserstoff, Fluor, Chlor, Brom, gegebenenfalls substituiertes $C_1$-$C_6$-Alkyl, oder gegebenenfalls substituiertes $C_6$-$C_{10}$-Aryl stehen, |

$R^4$ für Wasserstoff, Fluor, Chlor, Brom, Cyano, gegebenenfalls substituiertes $C_1$-$C_6$-Alkyl, gegebenenfalls substituiertes $C_6$-$C_{10}$-Aryl oder gegebenenfalls substituiertes $C_1$-$C_6$-Alkoxycarbonyl steht,

A für gegebenenfalls substituiertes $C_1$-$C_4$-Alkylen steht,

Y für gegebenenfalls substituiertes $C_1$-$C_6$-Alkoxy, Amino, gegebenenfalls substituiertes $C_1$-$C_6$-Alkylamino, gegebenenfalls substituiertes $C_1$-$C_6$-Dialkylamino oder gegebenenfalls substituiertes $C_6$-$C_{10}$-Aryl steht, oder

Y gemeinsam mit dem Rest $R^1$ für gegebenenfalls substituiertes $C_1$-$C_4$-Alkylen steht,

n für 0 oder 1 steht,

X für Wasserstoff, Fluor, Chlor, Brom oder gegebenenfalls substituiertes $C_1$-$C_4$-Alkyl steht, und

m für 1, 2 oder 3 steht,

wobei für die in den Definitionen von $R^1$, $R^2$, $R^3$, $R^4$, X und Y genannten Alkyl- und Alkoxyreste, sowie für die Alkylenreste in der Definitionen von A und Y und für den Rest Alkoxycarbonylalkyl in der Definition von $R^1$ als Substituenten jeweils Fluor, Chlor, Brom, Hydroxy, Methoxy und Ethoxy, für die (Di)alkylaminoreste in der Definition von Y (Di)methylamino, (Di)ethylamino oder (Di)isopropylamino und für die Arylsubstituenten in den Definitionen von $R^1$, $R^2$, $R^3$, $R^4$ und Y jeweils Fluor, Chlor, Brom, $C_1$-$C_4$-Alkyl und $C_1$-$C_4$-Alkoxy in Frage kommen.

Besonders bevorzugt sind Verbindungen der Formel (I),
worin

$R^1$ für Wasserstoff, Fluor, Chlor, Brom, Methyl, Ethyl, n-Propyl, i-Propyl, n-Butyl, s-Butyl, i-Butyl, t-Butyl, Phenyl, Naphthyl, Methoxycarbonylmethyl, Methoxycarbonylethyl, Ethoxycarbonylmethyl oder Ethoxycarbonylethyl steht,

$R^2$ für Wasserstoff, Fluor, Chlor, Brom, Methyl, Ethyl, Phenyl oder Naphthyl steht,

$R^3$ für Wasserstoff, Fluor, Chlor, Brom, Methyl oder Ethyl steht,

$R^4$ für Wasserstoff, Fluor, Chlor, Brom, Cyano, Methyl, Ethyl, Phenyl, Methoxycarbonyl oder Ethoxycarbonyl steht,

A für $-CH_2-$ oder $-CH_2CH_2-$ steht,

Y für Methoxy, Ethoxy, Amino, jeweils gegebenenfalls durch $C_1$-$C_4$-Alkylamino substituiertes (Di)-methylamino oder (Di)ethylamino oder für gegebenenfalls durch Methoxy substituiertes Phenyl oder Naphthyl steht, oder

Y gemeinsam mit dem Rest $R^1$ für $-CH_2-$ oder $-CH_2CH_2-$ steht,

n für 0 oder 1 steht,

X für Wasserstoff, Chlor, Brom, Methyl oder Ethyl steht, und

m für 1, 2 oder 3 steht.

Ganz besonders bevorzugt sind Verbindungen der Formel (I), in welchen

$R^1$ für Wasserstoff, Fluor, Chlor, Methyl oder Methoxycarbonylethyl steht,

$R^2$ für Wasserstoff oder Methyl steht,

$R^3$ für Wasserstoff,

$R^4$ für Wasserstoff,

A für $-CH_2-$ steht,

Y für Ethoxy, Amino oder 2-(N,N-Diisopropylamino)-ethylamino steht,

n für 1 steht, und

m für 3 steht.

Ebenfalls besonders bevorzugt sind Verbindungen der Formel (I),
in welchen

$R^1$ für Wasserstoff, Fluor, Chlor, Methyl oder Methoxycarbonylethyl steht,

$R^2$ für Wasserstoff oder Methyl steht,

$R^3$ für Wasserstoff,

$R^4$ für Wasserstoff,

Y für gegebenenfalls durch Methoxy substituiertes Phenyl steht, oder

Y gemeinsam mit dem Rest $R^1$ für $-CH_2CH_2-$ steht,

n für 0 steht, und

m für 3 steht.

Verwendet man beispielsweise 5-(Trifluormethyl)-5-methylpyrrolidin-2-on und 2-Bromessigsäureethylester als Ausgangsverbindungen, so läßt sich das erfindungsgemäße Verfahren zur Herstellung der Verbindungen (Ia) durch folgendes Formelschema darstellen:

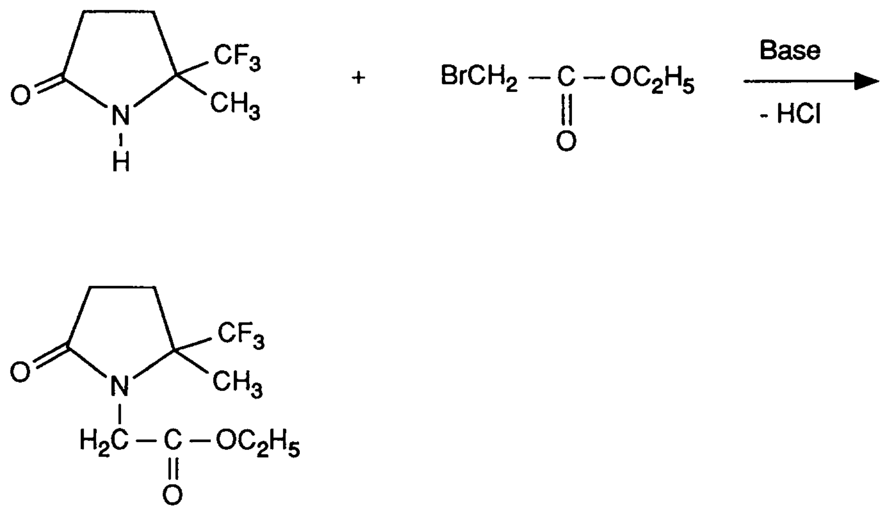


Die beim erfindungsgemäßen Verfahren zur Herstellung von Verbindungen der Formel (Ia) als Ausgangsstoffe verwendeten Pyrrolidinon-Derivate sind durch die Formel (II) allgemein definiert.

In Formel (II) haben $R^1$, $R^2$, $R^3$, $R^4$, X und m vorzugsweise bzw. insbesondere diejenige Bedeutung, die bereits oben im Zusammenhang mit der Beschreibung der Verbindungen der Formel (I) angegeben wurde.

Die Verbindungen der Formel (II) sind bekannt oder können in analoger Weise wie die bereits bekannten Verbindungen hergestellt werden (vgl. z.B. DE-A 4 029 054).

Verwendet man beispielsweise 5-(Trifluormethyl)-5-[(2-methoxycarbonyl)eth-1-yl]-pyrrolidin-2-on als Ausgangsverbindung, und setzt diese nacheinander mit Natronlauge, Salzsäure und Essigsäureanhydrid um, so läßt sich das erfindungsgemäße Verfahren zur Herstellung von Verbindungen der Formel (Ib) durch folgendes Syntheseschema darstellen:

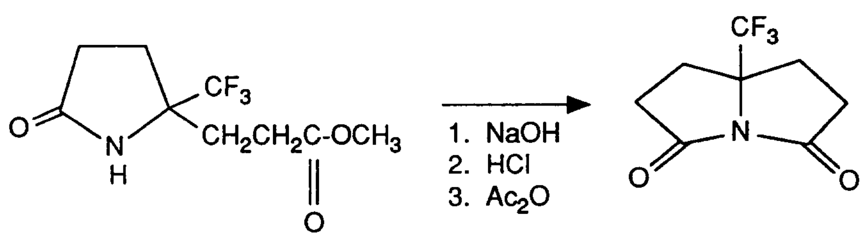


Die beim erfindungsgemäßen Verfahren zur Herstellung von Verbindungen (Ib) als Ausgangsstoffe verwendeten Pyrrolidinone der Formel (IV) sind durch die Formel (IV) allgemein definiert. In Formel (IV) haben die Reste $R^2$, $R^3$, $R^4$, X und m vorzugsweise bzw. insbesondere die bereits oben für Verbindungen der Formel (I) genannten Bedeutungen.

Die Verbindungen der Formel (IV) sind neu und ebenfalls Gegenstand der vorliegenden Erfindung.

Verwendet man beispielsweise 2-[5-(Trifluormethyl)-5-methylpyrrolidin-2-on]ethylacetat und Ammoniak als Ausgangsverbindungen, so läßt sich das erfindungsgemäße Verfahren zur Herstellung von Verbindungen der Formel (Ic) durch folgendes Formelschema darstellen:

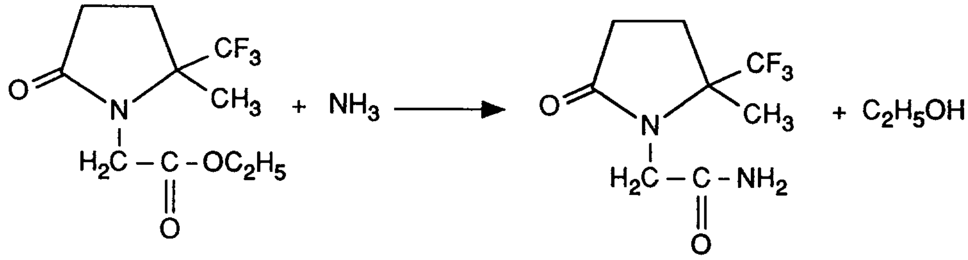

Die zur Herstellung von Verbindungen der Formel (Ic) verwendeten Verbindungen der Formel (Id) sind neue erfindungsgemäße Verbindungen und sind nach dem für die Verbindungen (Ia) angegebenen Verfahren erhältlich.

Verwendet man beispielsweise 2-[5-(Trifluormethyl)-5-methylpyrrolidin-2-on]-ethylacetat und N,N-Diisopropylethylendiamin als Ausgangsverbindungen, so läßt sich das erfindungsgemäße Verfahren zur Herstellung von Verbindungen der Formel (Ic) durch folgendes Formelschema darstellen:

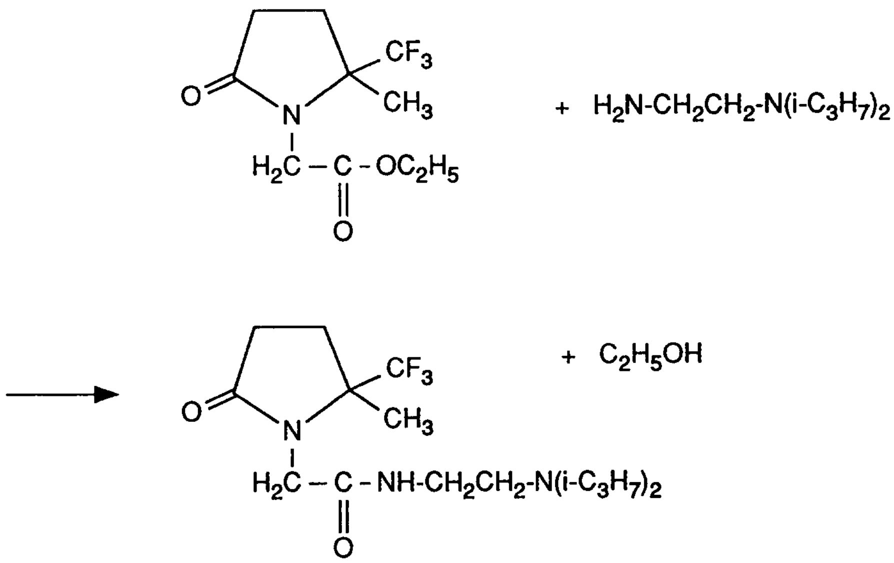

Die Herstellung der erfindungsgemäßen N-substituierten α-Fluoralkyl-Lactame der Formel (Ia) kann in Gegenwart eines Verdünnungsmittels erfolgen. Als Verdünnungsmittel kommen alle inerten organischen Lösungsmittel in Frage. Hierzu gehören insbesondere aliphatische und aromatische, wie Benzin, Benzol, Toluol, Xylol, Methylenchlorid, Chloroform, Tetrachlorkohlenstoff, Chlorbenzol und o-Dichlorbenzol, Ether, wie Diethylether, Diisopropylether, Dioxan und Tetrahydrofuran, Ketone, wie Aceton, Butanon, Methylisopropyl- und Methylisobutylketon, Nitrile, wie Acetonitril und Propionitril sowie die hochpolaren Lösungsmittel Dimethylsulfoxid und Hexamethylphosphorsäuretriamid.

Das erfindungsgemäße Verfahren zur Herstellung der Verbindungen (Ia) wird vorzugsweise in Gegenwart eines geeigneten Säurebindemittels durchgeführt. Als solche kommen alle üblicherweise verwendeten anorganischen und organischen Basen in Frage. Vorzugsweise verwendet man Alkalimetallalkoholate wie Natrium- oder Kalium-tert-butylat, Natrium- oder Kalium-tert-amylat, Alkalimetallcarbonate wie beispielsweise Natriumcarbonat, Kaliumcarbonat und Natriumhydrogencarbonat, ferner niedere tertiare Alkylamine, Cycloalkylamine oder Arylalkylamine, wie beispielsweise Triethylamin, N,N-Dimethyl-benzylamin, weiterhin Pyridin sowie 1,5-Diazabicyclo(4,3,0)-non-5-en und 1,4-Diazabicyclo(2,2,2)-octan.

Die Reaktionstemperaturen können bei dem erfindungsgemäßen Verfahren zur Herstellung der Verbindungen (Ia) in einem größeren Bereich variiert werden. Im allgemeinen arbeitet man bei Temperaturen zwischen -30 °C und 200 °C, vorzugsweise bei Temperaturen zwischen 0 °C und 130 °C.

Bei der Durchführung des erfindungsgemäßen Verfahrens zur Herstellung der Verbindungen (Ia) wird die Umsetzung von Verbindungen (II) mit (III) unter Verwendung eines der oben genannten Säurebindemittel, gegebenenfalls in Gegenwart eines Phasentransferkatalysators, wie z.B. quarternäre Ammonium- oder Phosphoniumsalze, in einem der oben genannten Verdünnungsmitteln durchgeführt. Das Reaktionsgemisch wird mehrere Stunden bei der erforderlichen Temperatur gerührt.

Die Aufarbeitung der Reaktionsmischung und die Isolierung der erfindungsgemäßen Reaktionsprodukte der Formel (Ia) erfolgt in allgemein üblicher Art und Weise.

Die Herstellung der erfindungsgemäßen N-substituierten α-Fluoralkyl-Lactame der Formel (Ib) erfolgt durch Umsetzung von Verbindungen der Formel (IV) mit einer Base.

Als Base kommen hiefür beispielsweise Alkalimetallhydroxide wie Natrium- oder Kaliumhydroxid, Alkalimetallcarbonate wie beispielsweise Natriumcarbonat, Kaliumcarbonat und Natriumhydrogencarbonat in Frage. Vorzugsweise verwendet man Alkalihydroxide wie Natrium- oder Kaliumhydroxid. Dabei wird die Base im allgemeinen in einer Menge von 1 bis 10 mol, vorzugsweise von 1 bis 5 mol, pro Mol der Verbindung der Formel (IV), eingesetzt.

Für die anschließende Umsetzung mit einer wasserbindenden Substanz kommen folgende Substanzen in Frage: Dicyclohexylcarbodiimid, N,N'-Carbonyldiimidazol, N-Ethoxycarbonyl-2-ethoxy-1,2-dihydroquinolin, $BF_3$-Etherat, $POCl_3$, $TiCl_4$, aliphatische und aromatische Säureanhydride, wie z.B. Essigsäureanhydrid, Propionsäureanhydrid. Bevorzugt wird Essigsäureanhydrid verwendet.

Das erfindungsgemäße Verfahren zur Herstellung der Verbindungen (Ib) wird im allgemeinen in Gegenwart eines Verdünnungsmittels durchgeführt. Als Verdünnungsmittel kommen dabei die bereits oben für die Herstellung der Verbindungen (Ia) genannten Verdünnungsmittel in Frage. Vorzugsweise verwendet man aliphatische und aromatische Verdünnungsmittel, wie Benzin, Benzol, Toluol, Xylol, Methylenchlorid, Chloroform, Tetrachlorkohlenstoff, Chlorbenzol und o-Dichlorbenzol, Ether, wie Diethylether, Diisopropylether, Dioxan und Tetrahydrofuran, Ketone, wie Aceton, Butanon, Methylisopropyl- und Methylisobutylketon, Nitrile, wie Acetonitril und Propionitril sowie die hochpolaren Lösungsmittel Dimethylsulfoxid und Hexamethylphosphorsäuretriamid.

Die Reaktionstemperaturen können bei dem erfindungsgemäßen Verfahren zur Herstellung der Verbindungen (Ib) in einem größeren Bereich variiert werden. Im allgemeinen arbeitet man bei -30 bis 200 °C.

Zur Durchführung des erfindungsgemäßen Verfahrens zur Herstellung der Verbindungen (Ib) werden Verbindungen mit der Formel (IV) in Wasser oder in Mischungen aus Wasser und Alkohol mit einer Base versetzt und mehrere Stunden bei der erforderlichen Temperatur gerührt. Nach Entfernung des Lösungsmittels wird die freie Säure durch Zugabe einer anorganischen Säure, z.B. Salzsäure, und anschließender Trocknung erhalten. Der Ringschluß erfolgt durch Rühren mit einer wasserbindenden Substanz bei der erforderlichen Temperatur.

Die Aufarbeitung und Isolierung der erfindungsgemäßen Produkte (Ib) erfolgt in allgemein üblicher Art und Weise.

Die Herstellung der erfindungsgemäßen Verbindungen (Ic) kann in Gegenwart eines inerten Verdünnungsmittels erfolgen. Dabei kommen grundsätzlich die bereits oben bei der Herstellung der Verbindungen (Ia) allgemein und bevorzugt genannten Verdünnungsmittel in Frage.

Das erfindungsgemäße Verfahren zur Herstellung von Verbindungen (Ic) wird im allgemeinen bei Temperaturen zwischen -10 °C und 200 °C, vorzugsweise zwischen 20 °C und 150 °C durchgeführt.

Bei der Durchführung des erfindungsgemäßen Verfahrens wird das Amin der Formel (V) im allgemeinen in einer Menge von 1 bis 50 mol, vorzugsweise von 1 bis 20 mol, bezogen auf 1 mol der Verbindung der Formel (Id), eingesetzt.

Die Aufarbeitung und Isolierung der erfindungsgemäßen Produkte (Ic) erfolgt in allgemein üblicher Art und Weise.

Die Verbindungen der Formel (III) und die Amine der Formeln (V) und (VI) sind in der organischen Chemie allgemein bekannte Verbindungen.

Die erfindungsgemäßen Wirkstoffe der Formel (I) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide, geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beipielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola:
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea:
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea;
(Konidienform: Drechslera, Syn. Helminthosporium);
Cochliobolus-Arten, wie beispielsweise Cochliobolus sativus;
(Konidienform: Drechslera, Syn. Helminthosporium);
Uromyces-Arten, wie beispielsweise Uromyces appendiculatus;
Puccinia-Arten, wie beipielsweise Puccinia recondita:
Tilletia-Arten, wie beispielsweise Tilletia caries;

Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beipielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrarkheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Phytophthora an Tomaten sowie von Venturia-Arten an Äpfeln einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methyethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasformig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit und Diatomeenerde und synthetische Gesteinsmehle, wie hochdisperse Kieselsäure, Aluminiumoxid und Silikate; als feste Trägerstoffe für Granulate kommen in Frage; z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyethylen-Fettalkohole-Ether, z.B. Alkylarylpolyglykol-Ether, Alkylsulfonate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen 0,0000001 bis 95 Gew.-% Wirkstoff, vorzugsweise 0,0001 bis 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die

Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

2-[5-(Trifluormethyl)-5-methylpyrrolidin-2-on]ethylacetat

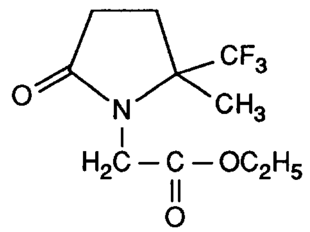

In 150 ml Toluol werden 33,5 g (0,2 mol) 5-(Trifluormethyl)-5-methylpyrrolidin-2-on suspendiert und nach Zugabe von 30 g (0,21 mol) gepulvertem Kaliumcarbonat, 2 g Tetrabutylammoniumbromid und 40 g (0,24 mol) 2-Bromessigsäureethylester ca 72 h am Rückfluß gerührt.

Durch GC-Kontrolle wird der Reaktionsverlauf verfolgt, eventuell werden nach ca. 48 h 5 g Kaliumcarbonat und gegebenenfalls 8 g 2-Bromessigsäureethylester nachdosiert.

Zur Aufarbeitung wird filtriert, das Salz mit Toluol nachgewaschen und die organische Phase im Wasserstrahlvakuum einrotiert. Das Rohmaterial (52 g) wird im Ölpumpenvakuum destilliert.

Es wurden 17 g Vorlauf vom Siedepunkt < 85°/0,02 Torr abgenommen, der lt. GC 65 % der oben angegebenen Verbindung enthält, sowie 28 g Hauptfraktion vom Siedepunkt 90-95°C/0,02 Torr, die lt. GC 95 % der gewünschten Verbindung enthält, was einer Gesamtausbeute von 77 % entspricht.

Die oben dargestellte Verbindung besitzt folgende NMR-Daten:

$^{1}$H-NMR (in $CDCl_3$/TMS): $\delta$ = 1,28 ppm; 1,49 ppm, 1,92-2,13 ppm, 2,34-2,68 ppm, 4,04 ppm und 4,19 ppm.

$^{19}$F-NMR (in $CDCl_3$/$CF_3COOH$): $\delta$ = -3,3 ppm.

Beispiel 2:

2-[5-(Trifluormethyl)-5-methylpyrrolidin-2-on]acetamid

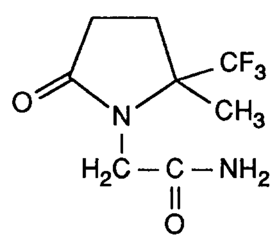

5 g (0,02 mol) 2-[5-(Trifluormethyl)-5-methylpyrrolidin-2-on]ethylacetat des Beispiels 1 werden 18 h bei Raumtemperatur in 10 ml konz. Ammoniak gerührt. Die Amidbildung kann per GC-Kontrolle verfolgt werden. Zur Aufarbeitung wird im Wasserstrahlvakuum eingeengt, es verbleibt ein hochviskoses Öl. Das Rohmaterial (4,2 g) wird im Ölpumpenvakuum destilliert.

Es wurden 3,4 g 2-[5-(Trifluormethyl)-5-methylpyrrolidin-2-on]acetamid vom Siedepunkt 135-138 °C/0,02 Torr entsprechend einer Ausbeute von 80 % der Theorie erhalten mit folgenden Daten:
$^1$H-NMR (in $d_6$-DMSO/TMS): δ = 1,42 ppm; 1,93-2,11 ppm, 2,21-2,42 ppm, 3,78 ppm und 7,05 bzw. 7,35 ppm.
$^{19}$F-NMR (in $d_6$-DMSO/$CF_3COOH$): δ = + 0,2 ppm.

Beispiel 3:

N-[2-[Bis(1-methylethyl)amino]ethyl-2-[5-(trifluormethyl)-5-methylpyrrolidin-2-on]acetamid

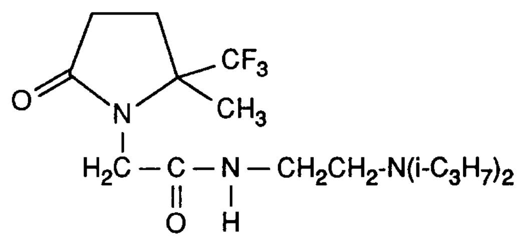

12,7 g (0,05 mol) 2-[5-(Trifluormethyl)-5-methylpyrrolidin-2-on]ethylacetat des Beispiels 1 werden 36 h bei 130 °C mit 8,6 g (0,06 mol) N,N-Diisopropylethylendiaminverrührt, dabei wird freigesetztes Ethanol abdestilliert. Anschließend wird im Ölpumpenvakuum destilliert.

Es wurden 5 g Vorlauf vom Siedepunkt < 110 °C/0,02 Torr abgenommen, der lt. GC 85 % der oben abgebildeten Verbindung enthielt, sowie eine Hauptfraktion vom Siedepunkt 140-145 °C/0,02 Torr, die lt. GC eine Reinheit von > 95 % aufwies, was einer Gesamtausbeute von 78 % entspricht
Die erhaltene Verbindung besitzt folgende NMR-Daten:
$^1$H-NMR (in $CDCl_3$/TMS): δ = 1,03 ppm; 1,53 ppm, 1,83-2,10 ppm, 2,32-2,54 ppm, 2,60 ppm, 3,03 ppm, 3,34 ppm, 3,97 ppm und ca 6,7 ppm.

Einige der Signale erscheinen zweifach aufgrund der Diastereotopie der Diisopropylaminogruppe.
$^{19}$F-NMR (in $CDCl_3$/$CF_3COOH$): δ = -0,3 ppm.

Die erhaltene Verbindung wird zur besseren Applizierbarkeit mit wässriger Salzsäure ins Hydrochlorid überführt.

Beispiel 4:

2-[5-(Trifluormethyl)-pyrrolidin-2-on]ethylacetat

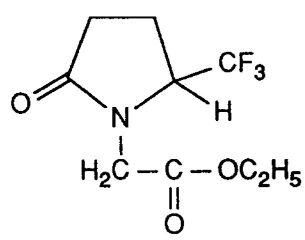

In 150 ml Toluol werden 30,6 g (0,2 mol) 5-(Trifluormethyl)-pyrrolidin-2-on suspendiert und nach Zugabe von 30 g (0,21 mol) gepulvertem Kaliumcarbonat, 2 g Tetrabutylammoniumbromid und 40 g (0,24 mol) 2-Bromessigsäureethylester ca. 36 h am Rückfluß gerührt. Durch GC-Kontrolle wird die Reaktion verfolgt, eventuell werden nach ca. 20 h 5 g Kaliumcarbonat und gegebenenfalls 8 g 2-Bromessigsäureethylester nachdosiert. Zur Aufarbeitung wird filtriert, das Salz mit Toluol nachgewaschen und die organische Phase im Wasserstrahlvakuum einrotiert. Das Rohmaterial (46,7 g) wird im Ölpumpenvakuum destilliert.

Es wurde ein Vorlauf vom Siedepunkt < 70 °/0,03 Torr erhalten, der lt. GC 63 % der oben dargestellten Verbindung enthielt, sowie eine Hauptfraktion, die lt. GC eine Reinheit von > 96% aufwies, was einer Gesamtausbeute von 74 % entspricht.

Die erhaltende Verbindung weist folgende NMR-Daten auf:
$^{1}$H-NMR (in $CDCl_3$/TMS): $\delta$ = 1,29 ppm; 2,11-2,72 ppm, 4,19 ppm und 4,28 ppm.
$^{19}$F-NMR (in $CDCl_3$/$CF_3COOH$): $\delta$ = + 3,05 ppm.

Beispiel 5:

N-[2-[Bis(1-methylethyl)amino]ethyl]-2-[5-(trifluormethyl)-pyrrolidin-2-on]-acetamid

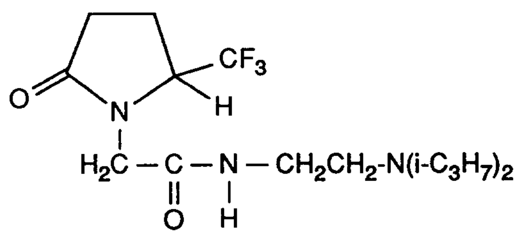

11,95 g (0,05 mol) 2[5-(Trifluormethyl)-pyrrolidin-2-on]ethylacetat des Beispiels 4 werden 36 h bei 130 ° C mit 8,6 g (0,06 mol) N,N-Diisopropylethylendiamin verrührt, dabei wird freigesetztes Ethanol abdestilliert. Anschließend wird im Ölpumpenvakuum destilliert.

Es wurde ein Vorlauf vom Siedepunkt < 115 ° C/0,02 Torr erhalten, der lt. GC die oben abgebildete Verbindung in einer Menge < 20 % enthielt, sowie eine Hauptfraktion vom Siedepunkt 132-137 ° C/0,02 Torr, die lt. GC eine Reinheit von > 90 % aufwies, was einer Gesamtausbeute von 79,5 % entspricht.

Die erhaltene Verbindung besitzt folgende NMR-Daten:
$^{1}$H-NMR (in $CDCl_3$/TMS): $\delta$ = 1,02 ppm; 2,12-2,54 ppm, 2,57 ppm, 3,02 ppm, 3,22 ppm, 4,08 ppm und 4,33 ppm.
$^{19}$F-NMR (in $CDCl_3$/$CF_3COOH$): $\delta$ = + 3,0 ppm.

Die erhaltene Verbindung wird zur besseren Applizierbarkeit mit wässriger Salzsäure ins Hydrochlorid überführt.

Beispiel 6:

2-[5-(Trifluormethyl)pyrrolidin-2-on]acetamid

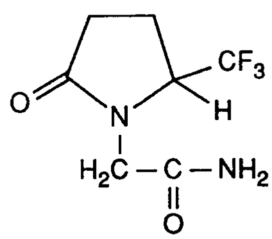

7,1 g (0,03 mol) 2-[5-(Trifluormethyl)pyrrolidin-2-on]ethylacetat des Beispiels 4 werden 21 h bei Raumtemperatur in 10 ml konz. Ammoniak gerührt. Es tritt rasche Niederschlagbildung auf, nach etwa 4 h ist das Reaktionsgemisch schwer rührbar, es werden weitere 5 ml $H_2O$ zugesetzt Der Feststoff wird anschließend in der Kälte abgesaugt, es werden 3,8 g reines Amid mit einem Fp.: 153-155 ° C isoliert.

Nach Einengen der Mutterlauge auf ca. 30 % werden weitere 1,3 g des Amids mit einem Fp.: 151-153 ° C gewonnen.
Ausbeute: 5,1 g entsprechend 81 % der Theorie.
$^{1}$H-NMR (in $CDCl_3$/TMS): $\delta$ = 1,97-2,10 ppm; 2,21-2,44 ppm, 3,91 ppm, 4,38 ppm und 7,1 bzw. 7,4 ppm.
$^{19}$F-NMR (in $d_6$-DMSO/$CF_3COOH$): $\delta$ = + 4,2 ppm.

Beispiel 7:

5-(Trifluormethyl)-1-azabicyclo[3.3.0]octan-2,8-dion

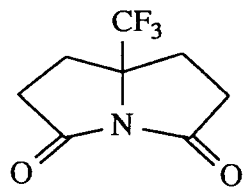

a) Verseifung
In 30 ml Methanol und 15 ml Wasser werden 12 g (0,05 mol) 5-(Trifluormethyl)-5-[(2-methoxycarbonyl)-eth-1-yl]-pyrrolidin-2-on und 4,5 g einer 50 %igen Natronlauge gemischt Nach Abklingen der exothermen Reaktion werden weitere 10 ml Wasser zugesetzt und 16 h am Rückfluß gerührt. Nach Abziehen der flüchtigen Bestandteile verbleiben 17,5 g des Natriumsalzes.
b) Freisetzung der Säure
Das Salz wird in 15 ml 20 %iger Salzsäure aufgenommen und 30 min gerührt. Anschließend wird zur Trockne gebracht. Es verbleiben 19 g Säure und Natriumchlorid.
c) Ringschluß
Der Feststoff wird unter Eiskühlung vorsichtig mit 10 ml Acetanhydrid versetzt. Nach Abklingen der Reaktion werden nochmals 20 ml Acetanhydrid zugesetzt und 16 h am Rückfluß gerührt.

Das Reaktionsgemisch wird heiß filtriert und das Filtrat im Wasserstrahlvakuum eingeengt Rohausbeute 10,5 g, die aus Toluol umkristallisiert werden. Es werden nach Filtration aus der Mutterlauge 9 g mit einem Fp. von 151-153 °C erhalten, die zur weiteren Reinigung entweder aus Isopropanol umkristallisiert oder im Hochvakuum bei 80-100 °C sublimiert werden.

Ausbeute nach Kristallisation aus Isopropanol 7,5 g, das sind 72 % der Theorie.
Fp.: 162-164 °C;MS:m/e = 207 ($M^+$).
$^1$H-NMR (in $CDCl_3$/TMS) $\delta$ = 2,08-2,27 ppm; 2,4-3,02 ppm.
$^{19}$F-NMR (in $CDCl_3/CF_3COOH$): $\delta$ = - 1,9 ppm
$^{13}$C-NMR (in $CDCl_3$/TMS) protonenentkoppelt: $\delta$ = 27,6 ppm; 33,9 ppm, 69,1 ppm, 127,1 ppm, 171,8 ppm.
IR (in KBr, nur charakteristische Banden der C=O-Gruppen):
$\tilde{\nu}$ = 1800 $cm^{-1}$ (st, br) und 1715 $cm^{-1}$ (m).

Beispiel 8:

N-[5-(Trifluormethyl)pyrrolidin-2-on]-4-methoxybenzoat

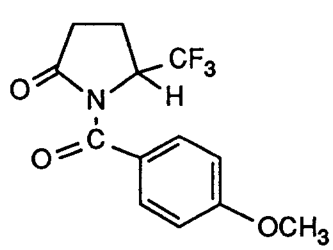

6,1 g (0,04 mol) 5-(Trifluormethyl)pyrrolidin-2-on werden in 10 ml Dioxan gelöst Nach Zusatz von 1,6 g Natriumhydroxid-Pulver werden unter Rühren innerhalb von 10 min 8,5 g (0,05 mol) Anisoylchlorid zugetropft. Die Reaktion ist leicht exotherm, es wird ca. 2 h nachgerührt.

Nach Einrühren in 50 ml Wasser wird die organische Phase, eventuell nach Zusatz von wenig Methylenchlorid abgetrennt und im Ölpumpenvakuum destilliert.

Es wurden 8,1 g Hauptfraktion vom Siedepunkt 159-162 °C/0,03 Torr erhalten, entsprechend einer Ausbeute von 70,4 % der Theorie.

Das Produkt erstarrt in der Vorlage, es weist einen Festpunkt von 80-82 °C auf.

$^1$H-NMR (in $CDCl_3$/TMS): δ = 2,21-3,03 ppm, 3,84 ppm, 5,28 ppm, 6,92 ppm und 7,71 ppm.

$^{19}$F-NMR (in $CDCl_3/CF_3COOH$): δ = + 1,7 ppm.

Beispiel 9:

N-[5-(Trifluormethyl)-5-methylpyrrolidin-2-on]-4-methoxybenzoat

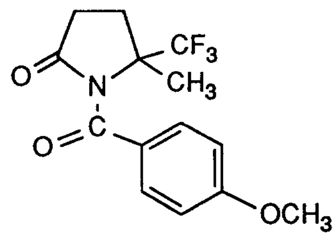

In 10 ml Dioxan werden 5,0 g (0,03 mol) 5-(Trifluormethyl)-5-methylpyrrolidin-2-on mit 3,3 g Triethylamin und 6,8 g (0,04 mol) Anisoylchlorid 16 h bei 110-120 °C verrührt. Der ausgefallene Feststoff wird abgesaugt und das Salz mit wenig Dioxan nachgewaschen. Die Dioxanlösung wird in 50 ml Wasser eingerührt und die organische Phase unter Zugabe von wenig Methylenchlorid abgetrennt. Nach Abziehen des Lösungsmittels verbleibt ein bräunlicher Feststoff, der aus Ligroin umkristallisiert wird.

Ausbeute: 8,0 g, das sind 88,6 % der Theorie.

Fp.: 94-97 °C

$^1$H-NMR (in $CDCl_3$/TMS): δ = 1,82 ppm, 2,02-2,18 ppm, 2,41-2,79 ppm, 3,68 ppm, 6,89 ppm und 7,63 ppm.

$^{19}$F-NMR (in $CDCl_3/CF_3COOH$): δ = + 0,8 ppm.

Beispiel 10:

N-[5-(Trifluormethyl)-5-(2-methyloxycarbonyleth-1-yl)pyrrolidin-2-on]-4-methoxybenzoat

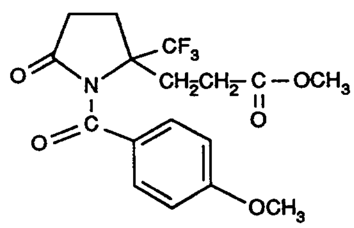

7,2 g (0,03 mol) 5-(Trifluormethyl)-5-(2-methyloxycarbonyleth-1-yl)pyrrolidin-2-on werden in 10 ml Dioxan gelöst mit 3,3 g Triethylamin und 6,8 g (0,04 mol) Anisoylchlorid 36 h bei 110-120 °C verrührt. Es bildet sich langsam ein weißlicher Niederschlag von $NEt_3$ • HCl. Zur Aufarbeitung wird abgesaugt und das Salz mit wenig Dioxan nachgewaschen. Die Dioxanlösung wird in 50 ml Wasser eingerührt und die organische Phase abgetrennt Es verbleibt ein braunes Öl.

Rohausbeute: 8,7 g (lt. $^1$H-NMR ca. 60 %ig)

Ausbeute: 58 % der Theorie

$^1$H-NMR (in $CDCl_3$/TMS): δ = 2,01-2,93 ppm, 3,64 ppm, 3,69 ppm, 6,88 ppm und 7,63 ppm.

$^{19}$F-NMR (in $CDCl_3/CF_3COOH$):δ = + 1,2 ppm.

Herstellung der Ausgangsverbindungen

Beispiel 11

5-(Trifluormethyl)-5-[(2-methoxycarbonyl)eth-1-yl]-pyrrolidin-2-on

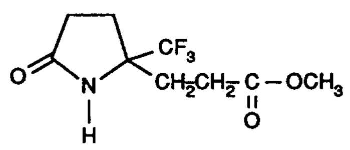

In einem 0,3 l V4A-Rührautoklaven werden 30,5 g (0,1 mol) 4-(Trifluormethyl)-4-nitropimelinsäure-dimethylester in 100 ml Methanol an 5 g 5 % Palladium auf Kohle bei 60-70 bar Wasserstoffdruck und 60 °C ca. 6 h hydriert. Nach Filtration wird das Methanol bei 40 °C im Wasserstrahlvakuum abgezogen, anschließend zur Vervollständigung der Lactambildung bei 70-80 °C im Wasserstrahlvakuum nachgerührt. Rohausbeute 23 g mit ca 85 %iger Reinheit (lt. GC, Rest freie Säure). Zur weiteren Reinigung wird im Ölpumpenvakuum destilliert.

Es wurden 19 g Hauptfraktion vom Siedepunkt 130-137 °C/0,03 Torr erhalten, entsprechend einer Ausbeute von 79 % der Theorie. 3 g Rückstand entsprach der freien Säure.
$^{1}$H-NMR (in $CDCl_3$/TMS): $\delta$ = 1,89-2,08 ppm, 2,18-2,68 ppm, 3,69 ppm und 7,16 ppm.
$^{19}$F-NMR (in $CDCl_3$/$CF_3COOH$): $\delta$ = - 2,0 ppm.

Beispiel 12:

4-(Trifluormethyl)-4-nitropimelinsäuredimethylester

In 150 ml Acetonitril werden bei 0-5 °C 27 g (0,465 mol) Kaliumfluorid (geglüht) und 86 g (1 mol) Acrylsäuremethylester vorgelegt und unter Rühren innerhalb von 60 min 60 g (0,465 mol) 2,2,2-Trifluornitroethan zugetropft. Die Reaktion ist merklich exotherm (bis ca. 60 °C). Nach Abkühlen auf Raumtemperatur wird 16 h nachgerührt. Der Ansatz wird filtriert, das Salz mit Acetonitril nachgewaschen und das Lösungsmittel und nicht umgesetztes Ausgangsmaterial am Rotationsverdampfer abgezogen.

Man erhält 108 g eines Öls mit ca. 92 %iger Reinheit (lt. GC), das im Ölpumpenvakuum destilliert wird.

Man erhält 6 g Vorlauf vom Siedepunkt < 120 °C/0,05 Torr, der weitgehend der Ausgangsverbindung entspricht, sowie 98 g Hauptfraktion vom Siedepunkt 124-127 °C/0,05 Torr entsprechend einer Ausbeute von 70 % der Theorie.
$^{1}$H-NMR (in $CDCl_3$/TMS): $\delta$ = 2,36-2,64 ppm, 3,71 ppm.
$^{19}$F-NMR (in $CDCl_3$/$CF_3COOH$): $\delta$ = + 7,5 ppm.
IR (Film; nur charakteristische Banden):
$\tilde{\nu}$ = 1745 $cm^{-1}$ (st, br, O=C-$OCH_3$); 1570 und 1355 $cm^{-1}$ (st, $NO_2$).

Anwendungsbeispiele

Beispiel A

Phytophthora-Test (Tomate)/protektiv

| | |
|---|---|
| Lösungsmittel: | 12,5 Gewichtsteile Aceton |
| Emulgator: | 0,3 Gewichtsteile Alkyl-aryl-polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigt in diesem Test z.B. die Verbindung gemäß Herstellungsbeispiel 6.

TABELLE A

Phytophthora - Test (Tomate) / protektiv

| Wirkstoff | Wirkungsgrad in % der unbehandelten Kontrolle bei einer Wirkstoffkonzentration von | |
|---|---|---|
| 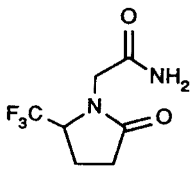 | 0,025 | 67 |

Beispiel B

Venturia-Test (Apfel)/protektiv

| | |
|---|---|
| Lösungsmittel: | 12,5 Gewichtsteile Aceton |
| Emulgator: | 0,3 Gewichtsteile Alkyl-aryl-polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca 70 % aufgestellt.

12 Tage nach der Inokulation erfolgt die Auswertung.

Eine deutliche Überlegenheit in der Wirksamkeit gegenüber dem Stand der Technik zeigt in diesem Test z.B. die Verbindungen gemäß Herstellungsbeispiele 5 und 6.

TABELLE B

Venturia - Test (Apfel) / protektiv

| Wirkstoff | Wirkungsgrad in % der unbehandelten Kontrolle bei einer Wirkstoffkonzentration von | |
|---|---|---|
| 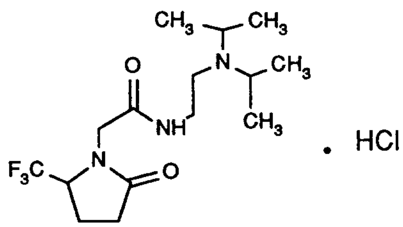 | 0,025 | 67 |
| 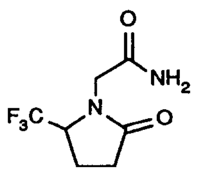 | 0,025 | 89 |

## Patentansprüche

**1.** α-Fluoralkyl-Lactame der Formel

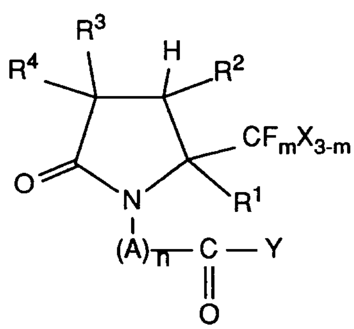

(I),

worin

| | |
|---|---|
| $R^1$ | für Wasserstoff, Halogen, Cyano, Alkyl, Aryl oder Alkoxycarbonyl steht, |
| $R^2$ und $R^3$ | unabhängig voneinander für Wasserstoff, Halogen, Alkyl oder Aryl stehen, |
| $R^4$ | für Wasserstoff, Halogen, Cyano, Alkyl, Aryl oder Alkoxycarbonyl steht, |
| A | für Alkylen steht, |
| Y | für Alkoxy, Amino, (Di)alkylamino oder Aryl steht, |
| | oder |
| Y | gemeinsam mit dem Rest $R^1$ für Alkylen steht, |
| n | für 0 oder 1 steht, |
| X | für Wasserstoff, Halogen oder Alkyl steht, und |
| m | für 1, 2 oder 3 steht. |

**2.** Verbindungen gemäß Anspruch 1,

worin

| | |
|---|---|
| $R^1$ | für Wasserstoff, Fluor, Chlor, Brom, gegebenenfalls substituiertes $C_1$-$C_6$-Alkyl, gegebenenfalls substituiertes $C_6$-$C_{10}$-Aryl oder gegebenenfalls substituiertes $C_1$-$C_6$-Alkoxycarbonyl($C_1$-$C_4$)-alkyl steht, |
| $R^2$ und $R^3$ | unabhängig voneinander für Wasserstoff, Fluor, Chlor, Brom, gegebenenfalls substitu- |

iertes $C_1$-$C_6$-Alkyl, oder gegebenenfalls substituiertes $C_6$-$C_{10}$-Aryl stehen,

$R^4$ für Wasserstoff, Fluor, Chlor, Brom, Cyano, gegebenenfalls substituiertes $C_1$-$C_6$-Alkyl, gegebenenfalls substituiertes $C_6$-$C_{10}$-Aryl oder gegebenenfalls substituiertes $C_1$-$C_6$-Alkoxycarbonyl steht,

A für gegebenenfalls substituiertes $C_1$-$C_4$-Alkylen steht,

Y für gegebenenfalls substituiertes $C_1$-$C_6$-Alkoxy, Amino, gegebenenfalls substituiertes $C_1$-$C_6$-Alkylamino, gegebenenfalls substituiertes $C_1$-$C_6$-Dialkylamino oder gegebenenfalls substituiertes $C_6$-$C_{10}$-Aryl steht, oder

Y gemeinsam mit dem Rest $R^1$ für gegebenenfalls substituiertes $C_1$-$C_4$-Alkylen steht,

n für 0 oder 1 steht,

X für Wasserstoff, Fluor, Chlor, Brom oder gegebenenfalls substituiertes $C_1$-$C_4$-Alkyl steht, und

m für 1, 2 oder 3 steht,

wobei für die in den Definitionen von $R^1$, $R^2$, $R^3$, $R^4$, X und Y genannten Alkyl- und Alkoxyreste, sowie für die Alkylenreste in der Definitionen von A und Y und für den Rest Alkoxycarbonylalkyl in der Definition von $R^1$ als Substituenten jeweils Fluor, Chlor, Brom, Hydroxy, Methoxy und Ethoxy, für die (Di)alkylaminoreste in der Definition von Y (Di)methylamino, (Di)ethylamino oder (Di)isopropylamino und für die Arylsubstituenten in den Definitionen von $R^1$, $R^2$, $R^3$, $R^4$ und Y jeweils Fluor, Chlor, Brom, $C_1$-$C_4$-Alkyl und $C_1$-$C_4$-Alkoxy in Frage kommen.

**3.** Verbindungen gemäß Ansprüchen 1 und 2, worin

$R^1$ für Wasserstoff, Fluor, Chlor, Brom, Methyl, Ethyl, n-Propyl, i-Propyl, n-Butyl, s-Butyl, i-Butyl, t-Butyl, Phenyl, Naphthyl, Methoxycarbonylmethyl, Methoxycarbonylethyl, Ethoxycarbonylmethyl oder Ethoxycarbonylethyl steht,

$R^2$ für Wasserstoff, Fluor, Chlor, Brom, Methyl, Ethyl, Phenyl oder Naphthyl steht,

$R^3$ für Wasserstoff, Fluor, Chlor, Brom, Methyl oder Ethyl steht,

$R^4$ für Wasserstoff, Fluor, Chlor, Brom, Cyano, Methyl, Ethyl, Phenyl, Methoxycarbonyl oder Ethoxycarbonyl steht,

A für $-CH_2-$ oder $-CH_2CH_2-$ steht,

Y für Methoxy, Ethoxy, Amino, jeweils gegebenenfalls durch $C_1$-$C_4$-Alkylamino substituiertes (Di)methylamino oder (Di)ethylamino oder für gegebenenfalls durch Methoxy substituiertes Phenyl oder Naphthyl steht, oder

Y gemeinsam mit dem Rest $R^1$ für $-CH_2-$ oder $-CH_2CH_2-$ steht,

n für 0 oder 1 steht,

X für Wasserstoff, Chlor, Brom, Methyl oder Ethyl steht, und

m für 1, 2 oder 3 steht.

**4.** Verbindungen gemäß Ansprüchen 1 bis 3, worin

$R^1$ für Wasserstoff, Fluor, Chlor, Methyl oder Methoxycarbonylethyl steht,

$R^2$ für Wasserstoff oder Methyl steht,

$R^3$ für Wasserstoff,

$R^4$ für Wasserstoff,

A für $-CH_2-$ steht,

Y für Ethoxy, Amino oder 2-(N,N-Diisopropylamino)-ethylamino steht,

n für 1 steht, und

m für 3 steht.

**5.** Verbindungen gemäß Ansprüchen 1 bis 3, in welchen

$R^1$ für Wasserstoff, Fluor, Chlor, Methyl oder Methoxycarbonylethyl steht,

$R^2$ für Wasserstoff oder Methyl steht,

$R^3$ für Wasserstoff,

$R^4$ für Wasserstoff,

Y für gegebenenfalls durch Methoxy substituiertes Phenyl steht, oder

Y gemeinsam mit dem Rest $R^1$ für $-CH_2CH_2-$ steht,

n für 0 steht, und

m für 3 steht.

**6.** Verbindungen der Formel

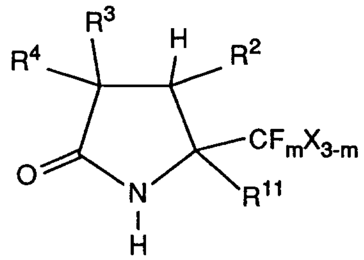

(IV),

worin

| | |
|---|---|
| $R^{11}$ | für Alkoxycarbonylalkyl steht, |
| $R^2$ und $R^3$ | unabhängig voneinander für Wasserstoff, Halogen, Alkyl oder Aryl stehen, |
| $R^4$ | für Wasserstoff, Halogen, Cyano, Alkyl, Aryl oder Alkoxycarbonyl steht, |
| X | für Wasserstoff, Halogen oder Alkyl steht, und |
| m | für 1, 2 oder 3 steht. |

**7.** Verbindungen gemäß Anspruch 6, worin

| | |
|---|---|
| $R^{11}$ | für gegebenenfalls substituiertes $C_1$-$C_4$-Alkoxycarbonyl-$C_1$-$C_4$-alkyl steht, |
| $R^2$ und $R^3$ | unabhängig voneinander für Wasserstoff, Fluor, Chlor, Brom, gegebenenfalls substituiertes $C_1$-$C_6$-Alkyl, oder gegebenenfalls substituiertes $C_6$-$C_{10}$-Aryl stehen, |
| $R^4$ | für Wasserstoff, Fluor, Chlor, Brom, Cyano, gegebenenfalls substituiertes $C_1$-$C_6$-Alkyl, gegebenenfalls substituiertes $C_6$-$C_{10}$-Aryl oder gegebenenfalls substituiertes $C_1$-$C_6$-Alkoxycarbonyl steht, |
| X | für Wasserstoff, Fluor, Chlor, Brom oder gegebenenfalls substituiertes $C_1$-$C_4$-Alkyl steht, und |
| m | für 1, 2 oder 3 steht, |

wobei für die in den Definitionen von $R^2$, $R^3$, $R^4$ und X genannten Alkyl- und Alkoxyreste und für den Rest Alkoxycarbonylalkyl in der Definition von $R^{11}$ als Substituenten jeweils Fluor, Chlor, Brom, Hydroxy, Methoxy und Ethoxy und für die Arylsubstituenten in den Definitionen von $R^2$, $R^3$ und $R^4$ jeweils Fluor, Chlor, Brom, $C_1$-$C_4$-Alkyl und $C_1$-$C_4$-Alkoxy in Frage kommen.

**8.** Fungizide Mittel, gekennzeichnet durch einen Gehalt an mindestens einem α-Fluoralkyl-Lactam der Formel (I) gemäß Ansprüchen 1 bis 5.

**9.** Verfahren zur Bekämpfung unerwünschter Pilze, dadurch gekennzeichnet, daß man mindestens ein α-Fluoralkyl-Lactam der Formel (I) gemäß Ansprüchen 1 bis 5 auf Pilze und/oder ihren Lebensraum einwirken läßt.

**10.** Verwendung von α-Fluoralkyl-Lactamen der Formel (I) gemäß Ansprüchen 1 bis 5 zur Bekämpfung von unerwünschten Pilzen.

**11.** Verfahren zur Herstellung von fungiziden Mitteln, dadurch gekennzeichnet, daß man mindestens ein α-Fluoralkyl-Lactam der Formel (I) gemäß Ansprüchen 1 bis 5 mit Streckmitteln und/oder oberflächenaktiven Mitteln vermischt.

Europäisches Patentamt

# EUROPÄISCHER RECHERCHENBERICHT

Nummer der Anmeldung
EP 94 10 0100

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| Y | EP-A-0 475 236 (BAYER AG) 18. März 1992<br>* Seite 7, Zeile 14 - Seite 7, Zeile 21; Ansprüche 1-9 *<br>--- | 1-11 | C07D207/26<br>C07D487/04<br>A01N43/36<br>A01N43/90<br>//(C07D487/04, 207:26,207:26) |
| Y | US-A-3 404 147 (GAF CORPORATION) 1. Oktober 1968<br>* Spalte 7, Zeile 9 - Spalte 7, Zeile 11; Ansprüche 1,2 *<br>--- | 1-11 | |
| A | EP-A-0 201 742 (SUNTORY LIMITED) 20. November 1986<br>* Ansprüche 1,2 *<br>----- | 1-11 | |

RECHERCHIERTE SACHGEBIETE (Int.Cl.5)
C07D
A61K

Der vorliegende Recherchenbericht wurde für alle Patentansprüche erstellt

| Recherchenort | Abschlußdatum der Recherche | Prüfer |
|---|---|---|
| MÜNCHEN | 7. Juni 1994 | Herz, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)